# EUROPEAN PATENT SPECIFICATION

(11) **EP 2 172 194 B1**
(45) Date of publication and mention of the grant of the patent: **20.10.2010**
(21) Application number: 09252233.3
(22) Date of filing: 18.09.2009
(51) Int. Cl.: A61K 9/70, A61K 31/27, A61K 31/137

(54) **Transdermal drug delivery system for liquid active ingredient**
Transdermales Arzneimittelabgabesystem für flüssige Aktivsubstanzen
Système d'administration transdermique de médicaments pour ingrédients actifs liquides

(30) Priority: 02.10.2008 EP 08253215
(43) Date of publication of application: 07.04.2010
(73) Proprietor: Amarin Technologies S.A., 1232 Buenos Aires (AR)
(72) Inventor: Forlano, Paula, 1232 Buenos Aires (AR); Scasso, Alejandro, 1232 Buenos Aires (AR); Stefano, Francisco, 1232 Buenos Aires (AR)
(74) Representative: Paget, Hugh Charles Edward

(56) References cited:
- WO-A-03/097020
- WO-A-2005/046600
- US-A1- 2006 240 087
- US-B1- 6 316 022
- US-B2- 7 070 808
- BODKIN J.A., AMSTERDAM J.D.: "Transdermal selegiline in major depression: a double-blind, placebo-controlled, parallel-group study in outpatients" AMERICAN JOURNAL OF PSYCHIATRY, vol. 159, no. 11, November 2002 (2002-11), pages 1869-1875, XP002515673
- DATABASE INTERNET [Online] 2009, XP002515698 retrieved from INTERNET accession no. http://www.merriam-webster.com/dictionary/ monolith
- DATABASE INTERNET [Online] 2006, XP002515699 retrieved from INTERNET accession no. http://www.dictionary.reference.com/search ?q=monol

## Description

### BACKGROUND

### Field of the invention:

The present invention is directed to a transdermal device containing a drug which is liquid at room temperature, selected from selegeline and rivastigmine and may be partially volatile at process temperature during manufacture of the device. In particular, the present invention seeks to provide a transdermal drug delivery device in which the loss of the drug during its manufacture is minimized while the adhesive and wearing properties are maintained.

### Description of related art:

Transdermal delivery of drugs provides many advantages over other routes of administration. Advantages of transdermal systems include convenience, non-interrupted therapy, improved patient compliance, reversibility of effects in case of adverse events (by removal of the system from the skin), elimination of "hepatic first pass" effect, high degree of control over blood concentration of any particular drug and consequent reduction of some side effects related to oral treatment.

Although transdermal systems have many advantages, drugs which are liquid at room temperature have several problems when being incorporated in those devices. One of the problems is a partial loss of the active drug during a drying step in the manufacture of the patches, which is usually performed between 50-100°C. Another problem related to many drugs which are liquid at 25°C is their plasticizing effect on the polymer matrix which results in the failure of adhesive and wearing properties.

Patents US 5462746 and EP 0593807, from LTS, disclose a patch for transdermal administration of volatile pharmaceutically active ingredients which provides a multi-element system to avoid loss of drug during its manufacture. The system has a first pressure sensitive adhesive (PSA) layer in which the volatile drug is loaded as a physiologically non-volatile acceptable salt thereof and a second PSA layer, with amino-group containing compound to liberate the free base from its salt.

Multi-layer systems always increase cost and have a more complex manufacture than monolayer systems.

Patent US 6316022, from Noven, discloses a transdermal composition for drugs which are liquid at room temperature. They found that if the composition is free of water and liquids which have a normal boiling point (a) optionally below processing temperatures and (b) greater than or equal to the temperature of the active drug, it does not suffer from the substancial loss of the active drug during production of a transdermal system. They also found that when polymers having a high shear resistance are used in the polymer matrix in a transdermal drug delivery system, the shear resistence of resulting polymer matrix is not significantly reduced due to plasticizing effect of liquid drugs.

High shear resistance polymers have the disadvantage of producing the so called "dry tack" phenomena consisting of a loss of adhesiveness immediately after the first contact of the adhesive with a due surface. This drawback, once produced, does not allow sticking the adhesive again. Patents US 7070808 and US 7150881, from Mylan, refer to transdermal devices of highly plasticizing drugs such as selegiline. As in US 6316022, they also disclose a system which is free of both water and liquids other than the active drug having a boiling point (i) below said predetermined processing temperature and (ii) equal to or greater than the boiling point of the active drug. In contrast to US 6316022, they disclose that a small class of acrylic based adhesive formulations can be used to maintain adequate properties with high plasticizing drugs; these acrylic polymeric adhesives have similar compositions. All the adhesives that they found with adequate performance include crosslinking agents, which can cause chemical interaction between the ingredients of the device. Example 15 of US 7150881 is a patch in which the adhesive has in itself a low shear value of one hour and has a shear value of 3.1 min. when 13% selegiline is incorporated. In Example 15, 5% selegiline and 10 % propylene glycol (PG) are present in the adhesive. The patch was unsatisfactory due to adhesive transfer.

The present invention provides transdermal devices containing an active pharmaceutical ingredient selected from rivastigmine and propargylamines such as selegeline.

Selegiline is a drug approved for the treatment of early stage Parkinson's disease, and depression. Other potential uses include senile dementia, narcolepsy, ADHD, and as nootropic and neuroprotective. In therapeutic doses it is a selective irreversible MAO-B inhibitor; however in larger doses it loses its specificity and also inhibits MAO-A. At dosages up to around 10 mg or so daily, selegiline retains its selectivity for the type-B MAO iso-enzyme; but it is also a weak reversible inhibitor of the type-A MAO iso-enzyme. Selegiline is used for the management of Parkinson, as an adjunct of levodopa/carbidopa, in doses of 10 mg daily by oral route (divided in two intakes of 5 mg each). Selegiline has been approved by transdermal route for the treatment of major depressive disorder in a dose range of 6 mg/24 h to 12 mg/24 h. The mechanism of action of transdermal selegiline as an antidepressant is not fully understood, but is presumed to be linked to potentiation of monoamine neurotransmitter activity in the CNS resulting from its inhibition of MAO activity. Rivastigmine is a reversible cholinesterase inhibitor (parasympathomimetic or cholinergic agent) that has been approved for the treatment of mild to moderate dementia of the Alzheimer's type, and mild to moderate dementia associated with Parkinson's disease. Rivastigmine has shown to be effective for the treatment of Alzheimer disease by oral route in a dose range of 6 to 12 mg per day, being the starting dose 1.5 mg twice a day. The oral dosage of rivastigmine shown to be effective in Parkinson's disease is 3 to 12 mg daily, given as twice a day intakes. The dose range for transdermal route varies between 4.6 mg to 9.5 mg daily. Patients who are on a total daily dose of < 6 mg of oral rivastigmine can be switched to 4.6 mg/24 h transdermally. Patients who are on a total daily dose of 6 to 12 mg of oral rivastigmine may be switched to 9.5 mg/24 h of transdermal rivastigmine. Pathological changes in dementia of the Alzheimer type and dementia associated with Parkinson's disease involve cholinergic neuronal pathways. While the precise mechanism of rivastigmine's action is unknown, it is postulated to exert its therapeutic effect by enhancing cholinergic function. This is accomplished by increasing the concentration of acetylcholine through reversible inhibition of its hydrolysis by cholinesterase.

### SUMMARY OF THE INVENTION

We have now found that certain drugs that are liquid at room temperature can be incorporated in a transdermal device without disadvantages previously mentioned.

The addition of certain non-volatile coadjuvants has now been found to reduce loss of the drug in the manufacturing process. Additionally and in contrast with what has been described previously, it has been found that it is possible to obtain suitable transdermal devices for active pharmaceutical ingredients that are in liquid state at 25°C using acrylic adhesives with moderate shear, high tack and without cross-linking agents.

One of the objectives of the present invention is to provide a patch for the transdermal administration of certain active pharmaceutical ingredients which are liquid at 25°C, which delivers a therapeutically acceptable dose of drug in a controlled way and which is simple and easy to manufacture and maintains its adhesive and wearing properties. We have surprisingly found that this could be obtained in a device with an acrylic PSA with moderate shear and without cross-linking agents.

Surprisingly, we could obtain by the present invention a transdermal device with a simple formulation, with few manufacturing steps, good adhesion properties and minimized loss of drug during its manufacture, which provides a therapeutically acceptable dose of drug. A further objective is to provide a device in which the non-drug components of the matrix have minimal or low chemical interaction with the drug, thus maximising drug availability.

According to the invention there is provided a monolithic device for transdermal administration of an active pharmaceutical ingredient which is liquid at 25°C, the device having an adhesive matrix layer which includes:
said active pharmaceutical ingredient which is selected from selegeline and rivastigmine,
an acrylic polymer pressure sensitive adhesive, without cross-linker agent containing a metal atom, the adhesive having a shear value of between 1.5 and 15 hours, and
a non-volatile coadjuvant selected from squalene and triethylcitrate present in the layer an amount of 1 to 15wt%.

The active pharmaceutical ingredient is selected from selegeline rivastigmine. Selegeline is a propargylamine. Propargylamines are known a group of pharmaceutical compounds which include selegiline and related compounds such as pargyline and rasigiline. Typically the pharmaceutical ingredient is present as the free base.

The non-volatile coadjuvant is selected from squalene and triethylcitrate. Most preferably the non-volatile coadjuvant is squalene, in view of its minimal interaction with the pharmaceutical ingredient.

By acrylic polymer pressure sensitive adhesive, we mean polyacrylate adhesives which are polymers or copolymers of a monomer or monomers selected from acrylic acid esters and methacrylic acid esters. Other monomers, such as acrylic acid and vinyl acetate, may be present. Typically the amount of such other monomers is not more than 15wt%, preferably not more than 10wt%. The adhesive copolymer is typically supplied by the manufacturer in solution, but the solvent is excluded in the compositions of the adhesive layers given herein.

Preferably the acrylic polymer pressure sensitive adhesive has pendent carboxyl (-COOH) or hydroxyl (-OH) functional groups attached to the polymer chain, since such functional groups have been found beneficial in avoidance or reduction of skin irritation in devices of the invention.

Particularly a device in which the acrylic pressure sensitive adhesive has carboxyl functional groups has been found most suitable for control of the delivery of the active component.

Preferably the amount of the coadjuvant is in the range 2 to 10%. Excessive coadjuvant may lead to poor properties such as exudation and lack of homogeneity.

In one particularly preferred embodiment of the invention the adhesive matrix layer comprises:
selegiline as the active pharmaceutical ingredient, present at 7 - 13wt%, more preferably 8 - 12.5wt%, and
squalene as the non-volatile coadjuvant, present at 1 - 10wt%, more preferably 4 - 8wt%, and
wherein the acrylic polymer pressure sensitive adhesive has a shear value in the range of 1.5 to 3 hours and has carboxyl functional groups.

More preferably the adhesive matrix layer comprises:
selegiline as the active pharmaceutical ingredient, present at 10 - 12.5wt%, and
squalene as said non-volatile coadjuvant, present at 4 - 6wt%.

Another particularly preferred embodiment of the invention is a device in which the adhesive matrix layer comprises:
rivastigmine as the active pharmaceutical ingredient, present at 12 - 30wt%, more preferably 12 - 20wt%, and
squalene as the non-volatile coadjuvant, present at 1 - 10wt%,
and wherein the acrylic polymer pressure sensitive adhesive has a shear value in the range of 1.5 to 3 hours and has carboxyl groups.

Most preferably in this device the adhesive matrix layer comprises:
rivastigmine as the active pharmaceutical ingredient, present at 16 - 30wt%, more preferably 16 - 20wt%, and
squalene as the non-volatile coadjuvant, present at 2 - 5wt%.

The device of the invention typically and in conventional manner includes, together with the adhesive matrix layer, a backing film on a first face of the adhesive layer and a release liner on an opposite face of the adhesive layer. A monolithic device is one in which there is a single layer only of material containing the active pharmaceutical component.

The invention further provides a method of manufacturing a device of the invention as set out above, the method including:
compounding the active pharmaceutical ingredient, the pressure sensitive adhesive, and the non-volatile coadjuvant in order to obtain a homogeneous mixture casting the homogeneous mixture onto a release liner, drying the release liner and homogeneous mixture at a temperature between 50 and 100°C, to form an adhesive layer on the release liner, and
applying a backing film to the adhesive layer.

### Brief description of the drawings:

As the present invention is susceptible of being performed in several different ways, some preferred embodiments of the invention are described below. Nevertheless, it should be understood that the present disclosure should be considered as an exemplification of the principles of the invention but it is not directed to limit the invention to the examples specifically illustrated.

In the drawings:
Figure 1 is a schematic view of a transdermal delivery device according the present invention
Figure 2 and Figure 3 show the in vitro permeation of selegiline from several adhesive matrixes in Example 4.
Figure 4 shows the percent of selegiline loss after drying vs. the amount of coadjuvants (Example 7 ).
Figure 5 shows the percent of rivastigmine after drying vs. the amount of selected coadjuvants (Example 8).
Figure 6 shows selegiline release profile from a device of the present invention vs. a commercial product (Example 10).
Figure 7 shows selegiline bioavailability of two devices of different size according to the present invention (Example 11).

### DETAILED DESCRIPTION OF THE INVENTION

In the transdermal devices described in the present invention the active drug, which is liquid at 25°C, is distributed homogeneously in the selected adhesive polymeric matrix.

Those devices, manufactured with moderate shear adhesives, can have an adequate balance of adhesive properties even when they have a drug load up to 20%, or even up to 30%. Such balance of adhesive properties and drug load render a device suitable for therapeutic uses.

The use of adhesives with moderate shear and high tack allows a good adhesion performance to be obtained. High shear adhesives can potentially produce a "dry tack phenomena" which is avoided in our invention. Additionally, the exclusion of adhesives with high shear implies a better adhesiveness to the skin and a more comfortable product for use by patients. By the term "adhesives with moderate shear" we mean adhesives with a shear value more than 1.5 hours and less than 15 hours (holding power: 4 psi @72°F) for the plain adhesive (containing no additive).

By the term "adhesives with high tack" we mean adhesives with a loop tack value higher than 35 oz/in² (15 kPa) for the plain adhesive. According to FINAT* Test Methods (FTM) & Equipment Comments, FTM 9 - * FINAT is Féderation Internationale des fabricants et transformateurs d'Adhésifs et Thermocollants sur papiers et autres supports.

The exclusion of cross-linker agents containing metals, particularly transition metal cross-linking agents, which act as catalysts of several chemical reactions, such as esterification, transesterification, oxidation and addition, avoids the possibility of chemical interaction with the active ingredient of the device and the consequent loss of potency, impurity formation and stability problems that reduce the acceptability of the resulting product for human and animal use from a safety point of view. Preferably the adhesive matrix layer contains no component acting as a cross-linking agent for the acrylic polymer.

Suitable drugs for use in the present invention are rivastigmine and selegiline and related compounds as pargyline, rasagiline and structurally related propargylamines. Particularly preferred are rivastigmine and selegiline.

It was surprisingly found that the addition of certain non-volatile coadjuvants reduces the loss of active drug during its manufacture. Further the addition of selected coadjuvants to acrylic adhesives with shear value less than 15 hours (holding power: 4 psi @72°F) does not alter adhesive performance beyond an acceptable limit. Suitable coadjuvants for the present invention are squalene and triethylcitrate. Without intending to be restricted to any particular theoretical explanation, we understand that the non-volatile coadjuvant funtions as a competitor of the active drug for evaporation, so that its presence disminishes the loss of the drug.

Particularly, within the above-selected coadjuvants, squalene (SQ)and triethylcitrate (TEC), in an amount in the range 1 to 15wt%, preferably 2 to 15wt%, more preferably 2 to 10wt%, yield devices of comfortable size that maintain suitable adhesive performance that reduces the loss of drug during their manufacture.

Additionally, it was unexpectedly found that within adhesives with low to moderate shear, those with functional groups as -COOH or -OH produce lower skin irritation. This is specially important in the case of selegiline because it may produce skin irritation (see references 1 and 2).

Moreover, within the adhesives with moderate shear and functional groups as -COOH or -OH, the ones with -COOH functional groups allow a better control in the release of the drug than those with no functional groups or the ones with -OH functionality.

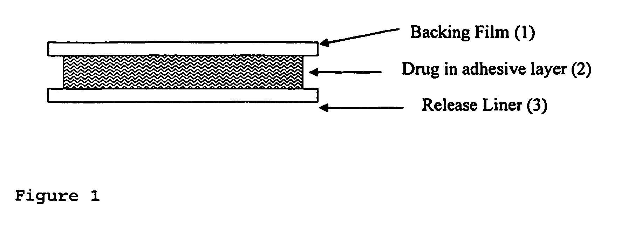

A typical device of the monolithic type of the present invention is shown in Figure 1. It includes a backing layer 1 that acts as a protection for the single adhesive layer 2. The adhesive layer 2 is composed of an acrylic polymer PSA containing the active drug and a non-volatile coadjuvant dissolved in it. On the other side of the adhesive layer 2 is the release liner 3 that is detached before the application of the device onto the skin.

One skilled in the art will be able to select the material for the backing layer to be used in the present invention. Examples, which are not limiting, of commercial backing layers at present are the following: polyethylene layer with a thickness between 25 to 100 µm (for example CoTran 9720 and CoTran 9711 from 3M), polyolefin layer with a thickness between 25 to 100 µm (for example CoTran 9722 from 3M), ethyl-vinyl acetate layer with a thickness between 25 to 100 µm (for example CoTran 9726 from 3M), pigmented polyester layer with a thickness between 25 to 100 µm (for example Scotchpack 9723 from 3M), ethyl-vinyl acetate layer with a thickness between 25 to 100 µm (for example CoTran 9702 and CoTran 9728 from 3M), polyolefin foam layer with a thickness between 250 to 2000 µm (for example Foam Tape 1777, Foam Tape 1779, Foam Tape 9751 y Foam Tape 9773), polyvinylchloride foam layer with a thickness between 250 to 2000 µm (for example Foam Tape 9772L), polyurethane layer with a thickness between 25 to 100 µm (for example Scotchpack 9701 from 3M), multilaminated of aluminised and pigmented polyester, polyethylene and ethyl-vinyl acetate with a thickness between 25 to 100 µm (for example Scotchpack 1006, Scotchpack 1009 and Scotchpack 1109 from 3M), multilaminated of polyester, polyethylene and ethyl-vinyl acetate with a thickness between 25 to 100 µm (for example Scotchpack 1220 from 3M), multilaminated of polyester and ethyl-vinyl acetate with a thickness between 25 to 100 µm (for example Scotchpack 9732 from 3M), cotton, polyester, rayon, nylon and polyurethane, woven and non-woven fabrics.

Likewise, as release liner can be used, among others, the following: siliconised polyester liner with a thickness between 25 to 250 µm (for example 1-5 PESTR 6200 P2, DCP-Lohja and Scotchpack 9742 from 3M), teflonated polyester liner with a thickness between 50 to 250 µm (for example Scotchpack 1022 from 3M) and a siliconised and aluminised polyester with a thickness between 50 to 250 µm (for example 1-3 MET-PESTR 6200 P2 DCP-Lohja).

The devices of the invention are preferably applied on those regions of the body far from mucosa or excessively keratinizated (such as palm of hand or sole of the foot).

### Description of the manufacturing process:

Basically, the manufacture of the present devices for transdermal administration, also known as drug-in-adhesive patches, involves the following steps:

The adhesive polymer (typically commercially available as solution of the polymer in a suitable solvent), the selected coadjuvant and the liquid active drug are compounded in a suitably sized vessel in order to obtain an homogeneous mixture that, once finished, is named coating mass.

The coating mass is cast onto a suitable film (generally it is the film acting later as release liner film), dried to eliminate all volatile compounds at temperature or temperatures in the range between 50 and 100°C and finally laminated to a suitable film (generally to the backing film).

These three unitary operations are performed in a continuous process that yield a drug in adhesive matrix sandwiched between the release liner and the backing film also called bulk laminate.

In a separate operation the bulk laminate is converted into patches of the desired size by die cutting or by other industrially acceptable process to finally be packed in suitable packaging material such as sachets, pouches, envelopes or blister packs.

### Definitions:

Holding power (shear adhesion): The ability of a tape to resist the static forces applied in the same plane as the backing. Usually expressed in a time required for a given weight per unit area to cause a given amount of tape to come loose from a vertical panel.

Peel adhesion: The force per unit width required to break the bond between a tape and a surface when peeled back, usually at 180°C at a standard rate and condition.

Throughout this specification, including the claims, the adhesive properties of shear adhesion and peel adhesion are measured employing the test methods adopted by the PSTC (Pressure Sensitive Tape Council) for Peel Adhesion (PSTC-1 Peel Adhesion of Single Coated Pressure Sensitive Tapes at 180° Angle) and Shear Adhesion (PSTC-7 Holding Power of Pressure Sensitive Tapes). The unit for peel adhesion is newtons/inch, and the unit for holding power (shear adhesion) is minutes. The test of holding power is performed under a load of 4 lbs/in² (28 kPa) and at 72°F (22°C).

Tack: The property of a pressure sensitive adhesive that allows it to adhere to a surface under very light pressure. It is determined by the ability of the adhesive to wet the surface contacted quickly.

This invention is further illustrated by the following examples. These examples should not be taken as limiting the scope of this invention in any manner. In these examples and throughout this specification and claims, percentages of components of the product are by weight of dry components (thus excluding the solvent of the adhesive polymer).

### EXAMPLES

### Example 1 (Reference example):

Adhesive performance of transdermal devices manufactured with acrylic adhesives with different values of shear, all of them without crosslinker agents and a load of 10% of selegiline base.

Transdermal devices used in this example were manufactured as described in "Description of the manufacturing process" above.

Adhesive properties were measured as described above. The plain adhesive values given are those of the dry adhesive polymer composition without added components.

The composition of each lot as well as the results of the adhesive experiments are shown in Table 1.

**Table 1: Composition of the lots and adhesive properties:**

| Lot | Adhesive (functionality) | Adhesive Properties of the plain adhesives | | | | Adhesive Properties of the devices | |
|---|---|---|---|---|---|---|---|
| | | Peel 180 (N/inch) | | Shear test (min) | | Peel 180 (N/inch) | Shear test (min) |
| | | Bibl.* | Exp.** | Bibl.* | Exp**. | | |
| Selsyn 15 | DT 87-4098 (-) | 7 | 4 | 600 | 722 | 10.2 | 51.9 |
| Selsyn 16 | DT 87-2287 (-OH) | 14 | > 25 | 60 | 23 | 19.3 Cohesive failure | 2.5 |
| Selsyn 17 | DT 87- 4287 (-OH) | 12 | 9.7 | 120 | 168 | 19.2 | 10.0 |
| Selsyn 18 | DT 87-2051 (-COOH) | 26 | > 25 | 7.5 | 2.6 | 9.9 Cohesive failure | 0.4 |
| Selsyn 19 | DT 87- 235^{a} (-COOH) | 15 | 11.9 | 120 | 114 | 6.0 | 64.8 |

| | | | | | | | |
|---|---|---|---|---|---|---|---|
| *data given by the manufacturer. **data measured in our laboratory. | | | | | | | |

DT numbers are the identifying numbers of acrylic polymer adhesives supplied by Durotak.

DT 87-235a and DT 87-2353 are acrylic polymers which can be used in the present invention. They contain no crosslinking agent. DT87-2353 is a random copolymer of 2-ethylhexylacrylate (2-EHA) 62.2%, methylacrylate (MA) 32.0%, glacial acrylic acid (GAA) 5.7% and glycidylmethacrylate (GMA) 0.03% in an organic solvent solution consisting of ethylacetate and hexane in the ratio 86.9 : 13.1. the copolymer is supplied at 35-38 % solid in solution.
DUROTAK 87-235A does not contain glycidylmethacrylate (GMA) 0.03% but is otherwise the same and has the same adhesive properties.

Conclusion: As expected, due to physicochemical characteristics of the active drug, selegiline reduces the cohesiveness in all the adhesives (measured by the shear test). Nevertheless, the adhesives tested, with a shear value for the plain adhesive more than 1.5 hours and less than 15 hours and without crosslinkers have an acceptable adhesive performance. The adhesives with shear value for the plain adhesive of 1 hour or less suffered cohesive failure.

Example 2 (Reference Example): Adhesive performance of transdermal devices manufactured with acrylic adhesives with different values of shear, all of them without crosslinker agents and a load of 20% or 30% of rivastigmine base.

Transdermal devices used in this example were manufactured as described in "Description of the manufacturing process" above.

The adhesive of each lot as well as the results of the adhesive experiments are shown in Table 2

**Table 2: Composition of the lots and adhesive properties:**

| Lot | Adhesive (functionality) | Drug load (%) | Adhesive Properties of the plain adhesives | | | | Adhesive Properties of the devices | |
|---|---|---|---|---|---|---|---|---|
| | | | Peel 180 (N/inch) | | Shear test (min) | | Peel 180 (N/inch) | Shear test (min) |
| | | | Bibl.* | Exp.** | Bibl.* | Exp**. | | |
| Rivasam 1 | DT 87-4098 (Non Functional) | 20 | 7 | 4 | 600 | 722 | 18.3 | 34.5 |
| Rivasam 2 | DT 87- 4287 (-OH) | | 12 | 9.7 | 120 | 168 | 25.7 | 25.5 |
| Rivasam 3 | DT 87- 235^{a} (-COOH) | | 15 | 11.9 | 120 | 111.3 | 11.5 | 42.3 |
| Rivasam 20 | DT 87-2287 (-OH) | | 14 | > 25 | 60 | 23 | 28.4 Cohesive failure | 1.8 |
| Rivasan 21 | DT 87-2051 (-COOH) | | 26 | > 25 | 7.5 | 2.6 | 13.9 Cohesive failure | 0.2 |
| Rivasam 25 | DT 87-4098 (Non Functional) | 30 | 7 | 4 | 600 | 722 | 23.5 | 27.9 |
| Rivasam 26 | DT 87- 4287 DT 87- 4287 (-OH) | | 12 | 9.7 | 120 | 168 | 29.1 | 3.5 |
| Rivasam 27 | DT 87- 235^{a} (-COOH) | | 15 | 11.9 | 120 | 111.3 | 15.6 | 32.6 |
| Rivasam 32 | DT 87- 2287 (-OH) | | 14 | > 25 | 60 | 23 | 48.4 Cohesive failure | 0.7 |
| Rivasam 33 | DT 87- 2051 (-COOH) | | 26 | > 25 | 7.5 | 2.6 | 18.1 Cohesive failure | 0.1 |

Conclusion: Rivastigmine reduces the cohesiveness in all the adhesives, like selegiline (Example. 1). In this case, also as with selegiline, all the adhesives tested, with a shear value for the plain adhesive more than 1.5 hours and less than 15 hours and without crosslinkers have an acceptable adhesive performance.

### Example 3 (Reference Example): Evaluation of dermal irritation in rabbits

Dermal irritation was tested using transdermal delivery devices manufactured as described in "Description of the manufacturing process" above with acrylic adhesives with different functionality and 15% of selegiline, all of them with shear value of more than 1.5 hours and less than 15 hours for the plain adhesive and without crosslinker agents (see Table 3). Each formulation was tested on 6 rabbits, with evaluations at 1, 2, 4, 6, 8 and 24 hs. The degree of adhesion and glue residue was also evaluated with skin reactions on an open-label 1-day-study. Rabbits belonging to the New Zealand Albino strain were used as animal model. According protocol, and after 14 days of acclimatization, the fur of back of 6 male rabbits was thoroughly shaved. Twenty four hours later one patch of each formulation of 10.6 cm² was glued in each rabbit to the shaved skin, according to a randomized schedule.

**Table 3: Composition of the lots**

| Lot | Adhesive type | Functionality |
|---|---|---|
| Selsyn 107 | DT 87-2353 | -COOH |
| Selsyn 108 | DT 87-4287 | -OH |
| Selsyn 109 | DT 87-4098 | -- |

Pure selegiline (25 µl) was used as control covered by Transpore tape (from 3M).

The irritation observed was classified according to the following scoring system:
Scale used for scoring erythema:

| | |
|---|---|
| 0: | No redness (erythema). |
| 1: | Barely perceptible redness. |
| 2: | Well defined redness. |
| 3: | Severe Erythema (beet redness) to slight eschar formation (injuries in depth). |

Reactions were scored as if they were covering the entire skin area treated (maximum score).

The Dermal Irritation Index (DII) was calculated as the the average of the individual data at each evaluation time.

Results are shown in Tables 4-7

**Table 4: Scores for Skin Erythema for lot SELSYN 107 (Patches 1 to 6)**

| Rabbit | 1h | 2h | 4h | 6h | 8h | 24h | DII |
|---|---|---|---|---|---|---|---|
| 1 | 0 | 1 | 1 | 1 | 22 | 1 | 1.0 |
| 2 | 0 | 0 | 0 | 0 | 0 | 0 | 0.0 |
| 3 | 0 | 0 | 0 | 0 | 0 | 0 | 0.0 |
| 4 | 0 | 0 | 0 | 0 | 0 | 1 | 0.2 |
| 5 | 0 | 0 | 0 | 0 | 0 | 0 | 0.0 |
| 6 | 0 | 0 | 0 | 0 | 0 | 0 | 0.0 |
| Erythema Score | *0.0* | *0.2* | *0.2* | *0.2* | *0.3* | *0.3* | **0.2** |

All patches of Lot 107 remained 100% adhered during the 24h using period, except for No.2 (70% adhered).

If patches are removed gently, they do not elicit redness by the mechanical strength exerted. No glue residue was left on the skin after patch removal.

**Table 5: Scores for Skin Erythema for lot SELSYN 108 (Patches 11 to 16)**

| Rabbit | 1h | 2h | 4h | 6h | 8h | 24h | DII |
|---|---|---|---|---|---|---|---|
| 1 | 0 | 0 | 0 | 0 | 1 | 1 | 0.3 |
| 2 | 0 | 0 | 0 | 0 | 0 | 1 | 0.2 |
| 3 | 0 | 0 | 0 | 0 | 1 | 2 | 0.5 |
| 4 | 0 | 0 | 0 | 0 | 0 | 1 | 0.2 |
| 5 | 0 | 0 | 0 | 0 | 0 | 0 | 0.0 |
| 6 | 0 | 0 | 0 | 0 | 0 | 0 | 0.0 |
| Erythema Score | *0.0* | *0.0* | *0.0* | *0.0* | *0.3* | *0.8* | **0.2** |

All patches of Lot 108 remained 100% adhered during the 24h using period.

If patches are removed gently, they transfer the adhesive to the skin and elicit some redness by the mechanical strength exerted.

An important quantity of glue residue was left on the skin after patch removal.

**Table 6: Scores for Skin Erythema for lot SELSYN 109 (Patches 21 to 26)**

| Rabbit | 1h | 2h | 4h | 6h | 8h | 24h | DII |
|---|---|---|---|---|---|---|---|
| 1 | 0 | 0 | 0 | 0 | 1 | 0 | 0.2 |
| 2 | 0 | 1 | 1 | 1 | 1 | 2 | 1.0 |
| 3 | 0 | 0 | 0 | 0 | 1 | 2 | 0.5 |
| 4 | 0 | 0 | 0 | 0 | 1 | 1 | 0.3 |
| 5 | 1 | 1 | 1 | 1 | 2 | 2 | 1.3 |
| 6 | 0 | 0 | 0 | 0 | 0 | 0 | 0.0 |
| Erythema Score | *0.2* | *0.3* | *0.3* | *0.3* | *1.0* | *1.2* | **0.6** |

All patches of Lot 109 remained 100% adhered during the 24h using period.

If patches are removed gently, they do not elicit redness by the mechanical strength exerted.

No glue residue was left on the skin after patch removal.

**Table 7: Scores for Skin Erythema for Control (pure selegiline 25 µl)**

| Rabbit | 1h | 2h | 4h | 6h | 8h | 24h | DII |
|---|---|---|---|---|---|---|---|
| 1 | 0 | 0 | 0 | 1 | 1 | 1 | 0.5 |
| 2 | 0 | 1 | 1 | 1 | 1 | 1 | 0.8 |
| 3 | 0 | 0 | 0 | 0 | 1 | 1 | 0.3 |
| 4 | 0 | 0 | 0 | 0 | 1 | 1 | 0.3 |
| 5 | 0 | 0 | 0 | 0 | 1 | 1 | 0.3 |
| 6 | 0 | 0 | 0 | 1 | 1 | 0 | 0.3 |
| Erythema Score | *0.0* | *0.2* | *0.2* | *0.5* | *1.0* | *0.8* | **0.4** |

The Transpore tape (from 3M), used to cover the selegiline poured onto the skin, did not develop skin reactions during 24 h of use in any of the rabbits tested.

The dermal irritation indexes obtained were interpreted following ISO/WD 10993-10:

| DII | Response category |
|---|---|
| 0-0.4 | negligible |
| 0.5-1.9 | slight |
| 2.0-4.9 | moderate |
| 5.0-8.0 | severe |

We conclude that lot 109, formulated with a non-functional acrylic adhesive produces the highest irritation, while the lots formulated with -COOH or -OH functional groups produce minimal to null irritation.

Example 4 (reference example): Comparative permeation of transdermal devices manufactured with acrylic adhesives with different functionality, all of them with shear value less than 15 hours for the plain adhesive and without cross-linker agents, 10% of selegiline base and 90% of adhesive.

The devices were manufactured as described in "Description of the manufacturing process" above.

The composition of the lots is shown in Table 8

**Table 8: Composition of the lots:**

| Lot | Adhesive | functionality |
|---|---|---|
| Selsyn 15 | DT 87-4098 | -- |
| Selsyn 17 | DT 87- 4287 | OH |
| Selsyn 19 | DT 87- 235A | COOH |

Commercial product Emsam 40 mg, lot 6P 0024, was also used for the permeation assays. Emsam contains 40mg/40cm² selegiline in acrylic adhesive.

Measurements were performed by triplicate using Valia-Chien type cells, with constant stirring in a water bath with a fixed temperature (34°C). Circular pieces of human female intact skin (stratum corneum, epidermis and dermis) with 2 cm diameter and 300 µm thickness were used. The solution used consisted of 5.5 ml of a phosphate buffer isotonic solution adjusted to pH = 6.0. Samples were collected at 2, 4, 8, 12, 24 and 48 hours after the start of the experiment and selegiline content was analysed by HPLC using a suitable technique.

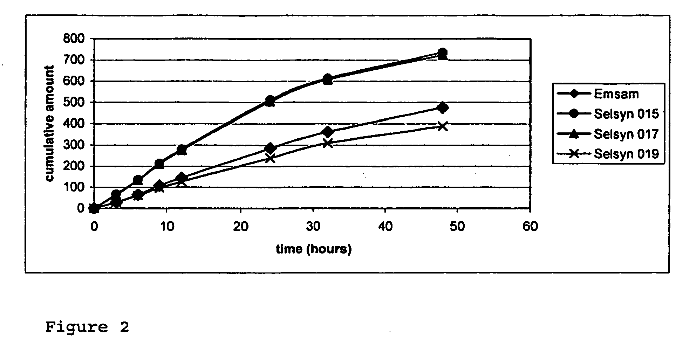

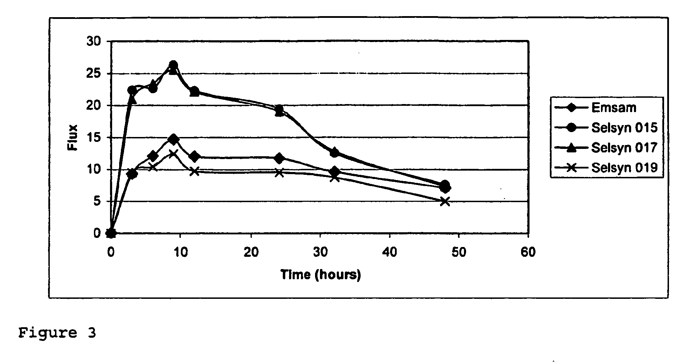

The results obtained are shown in Figs. 2 and 3

Example 5: Effect of non-volatile coadjuvants in patches formulated with an acrylic adhesive with shear value more than 1.5 hours and less than 15 hours for the plain adhesive and without crosslinker agents, and -COOH functionality (DT 87-2353).

### a) Effect of squalene

Lots with 10.5% of selegiline and different amounts of squalene were manufactured as described in "Description of the manufacturing process" above, and their general appearance was evaluated (visual and tactile inspection done to check lack of homogeneity, material exuding from the dry adhesive matrix, excessive softness, lack or excessive tack and other undesirable attributes). The composition of the lots and the results obtained are shown in Table 9.

**Table 9: Composition of lots with different amount of squalene and general evaluation of its appearance.**

| Lot | Squalene (%) | General appearance | | | |
|---|---|---|---|---|---|
| | | homogeneity | exudate | softness | tack |
| 090 | 2.0 | OK | OK | OK | OK |
| 091 | 5.5 | OK | OK | OK | OK |
| 092 | 11 | OK | OK | OK | OK |
| 093 | 15 | OK | OK | OK | OK |
| 094 | 20 | OK | unacceptable | | |

We conclude that the use of squalene as coadjuvant is suitable up to 15%.

### b) Effect of polyethylene glycol (PEG), triethylcitrate (TEC), Silicone 100, propylene glycol (PPG) and ethyl oleate (EO).

Lots with 12.5% of selegiline and different amounts of coadjuvants were manufactured as described in "Description of the manufacturing process" and were evaluated as in part a). The composition of the lots and the results obtained are shown in Table 10.

**Table 10: Composition of lots with different amount of coadjuvants and evaluation of its appearance.**

| Lot | Type of coadj. | Coadj. (%) | Appearance | | | |
|---|---|---|---|---|---|---|
| | | | homogeneity | exudate | softness | tack |
| 130 | PEG | 5 | OK | OK | Unacceptable | OK |
| 131 | | 10 | OK | Unacceptable | | |
| 132 | | 15 | OK | Unacceptable | | |
| 133 | TEC | 5 | OK | OK | OK | OK |
| 134 | | 10 | OK | OK | OK | OK |
| 135 | | 15 | OK | OK | OK | OK |
| 136 | Silicone fluid | 5 | OK | Unacceptable | OK | OK |
| 137 | | 10 | Unacceptable | | | |
| 138 | | 15 | Unacceptable | | | |
| 139 | PPG | 5 | OK | OK | OK | OK |
| 140 | | 10 | OK | OK | OK | OK |
| 141 | | 15 | OK | OK | Unacceptable | |
| 142 | EO | 5 | OK | OK | OK | OK |
| 143 | | 10 | OK | OK | OK | OK |
| 144 | | 15 | OK | OK | Unacceptable | |

We conclude that PEG and silicone fluid are not suitable as coadjuvants because they produce a marked modification of the rheological properties that affect severely the structural performance of the adhesive polymer.

PPG and EO are suitable up to 10% only. TEC is suitable up to 15%.

Example 6: Adhesive performance of patches with suitable coadjuvants.

Patches loaded with approximately 5% of different suitable coadjuvants according to Example 5 and selegiline base and a reference patch without coadjuvant were manufactured as described in "Description of the manufacturing process" above, using DT 87-2353 as the adhesive.

The coadjuvant used in each lot as well as the results of the adhesive experiments are shown in Table 11

**Table 11: Composition and adhesive properties for selegiline patches loaded with different coadjuvants**

| Batch | Selegiline base (%) | Coadjuvant (%) | Adhesive Properties | |
|---|---|---|---|---|
| | | | Peel 180 (N/inch) | Shear test (min) |
| Reference (plain adhesive) | -- | -- | 11.9 | 111.3 |
| Selsyn 133 | 12.5 | Triethylcitrate (5%) | 8.4 | 11.0 |
| Selsyn 120 | 12.5 | Squalene (5.5%) | 5.8 | 11.2 |

We conclude that these non-volatile liquids, as expected, alter the adhesive properties of the plain adhesives, but we consider that they are suitable because the products have a 180° peel value higher than 3 (N/inch) and a shear test value higher than 5 minutes.

Example 7: Effect of suitable non-volatile coadjuvants in loss of selegiline during manufacture.

Lots with DT 87-2353 as adhesive, selegiline and increasing amounts of squalene (SQ) and triethylcitrate (TEC) were manufactured as described in "Description of the manufacturing process" above and the amount of selegiline present after drying was measured. The amount of ingredients in the formulation and the loss of selegiline after drying in each lot are shown in Table 12.

**Table 12: Amount of ingredients in the formulation and percent of selegiline loss after drying**

| Lot | Initial % of selegiline | Coadjuvant | % of coadjuvant | Percent of selegiline loss |
|---|---|---|---|---|
| 121 | 12.5 | ----- | ----- | 12.8 |
| 098 | 10.5 | SQ | 5.5 | 10.5 |
| 092 | 10.5 | SQ | 11.0 | 7.6 |
| 093 | 10.5 | SQ | 15.0 | 3.8 |
| 133 | 12.5 | TEC | 5.0 | 9.2 |
| 135 | 12.5 | TEC | 15.0 | 5.0 |

Selegiline content was analysed by HPLC using a suitable technique

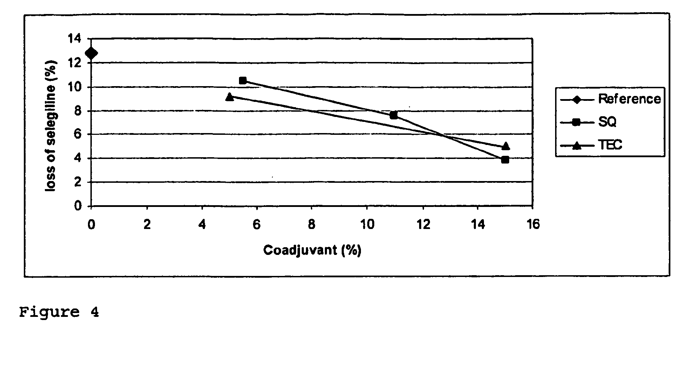

The percent of selegiline loss in the device after drying vs. the amount of coadjuvant is seen in Figure 4.

We conclude that the addition of squalene and triethylcitrate, up to 15%, diminishes the amount of active drug loss during drying in a concentration dependent manner.

Example 8: Effect of non-volatile coadjuvants in patches made with an acrylic adhesive having a shear value less than 15 hours for rivastigmine.

Lots with DT 87-2353 as adhesive, 20.0% of initial load of rivastigmine and increasing amounts of coadjuvants were manufactured as described in "Description of the manufacturing process" above and the amount of rivastigmine present after drying was measured. The amounts of coadjuvants in the formulation and of rivastigmine after drying in each lot are shown in Table 13.

**Table 13: Amount of coadjuvant in the formulation and of rivastigmine after drying.**

| Lot | Coadjuvant | Coadjuvant (%) | Percent of rivastigmine loss (after drying) |
|---|---|---|---|
| Rivasam 4 | --- | --- | 18.0 |
| Rivasam 5 | TEC | 5.0 | 16.0 |
| Rivasam 6 | | 10.0 | 12.0 |
| Rivasam 7 | | 15.0 | 5.5 |
| Rivasam 14 | Squalene | 5.0 | 9.5 |
| Rivasam 15 | | 10.0 | 6.0 |
| Rivasam 16 | | 15.0 | 4.0 |

Rivastigmine content was analysed by HPLC using a suitable technique

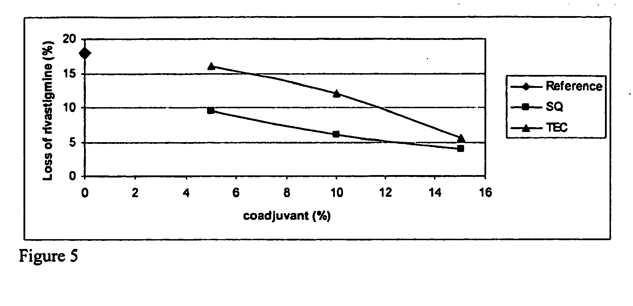

The percent of rivastigmine in the device after drying vs. the amount of coadjuvant is seen in Figure 5.

We conclude that the addition of selected coadjuvants, up to 15% diminishes the amount of active drug loss during drying in a concentration dependent manner.

### Example 9: Chemical interaction between selegiline and selected coadjuvants

Binary mixtures were prepared dissolving 1% of selegiline in the coadjuvant of interest. The mixtures were stored seven days at 40°C and related substances were quantified with suitable HPLC techniques in comparison with pure selegiline in the same conditions (control).

Table 14 shows chromatographic purity of selegiline in TEC and SQ and the percent of any secondary peak higher than 0.05%

**Table 14: Chemical interaction between selegiline and selected coadjuvants**

| Coadjuvant | Selegiline base | Impurity |
|---|---|---|
| TEC | 98.58% | rrt: 1.56 0.42% rrt: 2.09: 0.98% |
| Squalene | 99.89% | rrt: 2.04: 0.07%, |
| Control | 99.98% | < 0.05% |

| | | |
|---|---|---|
| rrt = relative retention time to selegiline peak. | | |

We conclude that selegiline base is stable to thermal treatment because no peaks higher than 0.05% were detected. Neither of the coadjuvants tested show high interaction with selegiline, although TEC shows more chemical interaction with selegiline than squalene.

Example 10: Evaluation of in vivo drug release profile and dermal irritation in rabbits

The in vivo release profile and the dermal irritation tests were performed using transdermal delivery devices manufactured as described in "Description of the manufacturing process" above with selegiline as active agent and the commercial device Emsam (lot 6PO0024).

### a) In vivo release profile

The in vivo release profile was estimated by measuring the selegiline left in the patch after its application to rabbits at 4, 8 and 24 hours of use.

Male rabbits belonging to the New Zealand Albino strain were used as animal model. According to the protocol, and after acclimatization, the fur of the back of the 6 rabbits was thoroughly shaved. Twenty four hours later, on each rabbit 3 patches of each formulation of 10.6 cm² were applied to the shaved skin, according to a randomization schedule. Water was offered ad libitum, but food was not allowed from 3h before patch application through to the patch removal (after 24 h of use), to avoid the influence of any potential tyramine effects.

Patches were removed at 4, 8 and 24 hours of use, and the worn patches were kept frozen until Selegiline remnant assays.

The formulations tested are in Table 15.

**Table 15: Formulation tested in the evaluation of in vivo release profile and termal irritation in rabbits**

| Formulation | Non active components | Selegiline content (%) | Selegiline content (mg/TDS) | Size |
|---|---|---|---|---|
| Selsyn 087-1 | Duro tak 87-2353 Squalene | 12.4 | 13.6 | 10.6 sqcm |
| EMSAM 40 mg/40cm² Lot 6P0024 (40 mg/40cm²) Cut to 10.6 cm² | Acrylic adhesive | 11.0 | 10.6 | 10.6 sqcm |

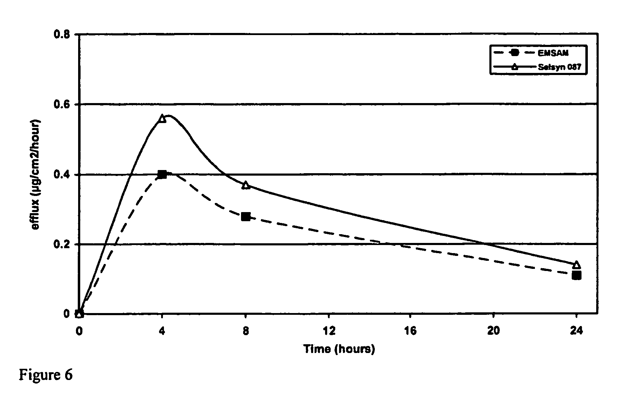

The results obtained are shown in Figure 6:

The results show that the patch with a formulation like the Selsyn 087 (a 12.5% Selegiline loaded COOH polymer containing 5.5% of squalene as non-volatile liquid), according to the present invention, has a selegiline release profile similar to the commercial product Emsam.

The fact that the patch according to the present invention, Selsyn 087, yielded higher release output has the advantage of allowing the possibility to design a smaller patch than the actual commercial one rendering the same selegiline release profile.

### b) Dermal irritation

The irritation observed was classified according to the following scoring system:

Scale used for scoring erythema:

| | |
|---|---|
| 0: | No redness (erythema). |
| 1: | Barely perceptible redness. |
| 2: | Well defined redness. |
| 3: | Severe Erythema (beet redness) to slight eschar formation (injuries in depth). |

Reactions were scored as if they were covering the entire skin area treated (maximum score).

The Dermal Irritation Index (DII) was calculated as the the average of the individual data at each evaluation time.

Results are shown in Tables 16-17

**Table 16: Scores for Skin Erythema for lot SELSYN 087 measured following patch removal**

| Rabbit | 4h | 8h | 24h | DII |
|---|---|---|---|---|
| 1 | 0 | 0 | 0 | 0.0 |
| 2 | 0 | 0 | 1 | 0.3 |
| 3 | 0 | 0 | 0 | 0.0 |
| 4 | 0 | 0 | 1 | 0.3 |
| 5 | 0 | 0 | 0 | 0.0 |
| 6 | 0 | 0 | 0 | 0.0 |
| Erythema Score | *0.0* | 0.0 | *0.3* | **0.1** |

**Table 17: Scores for Skin Erythema for Emsam (lot 6PO0024) measured following patch removal**

| Rabbit | 4h | 8h | 24h | DII |
|---|---|---|---|---|
| 1 | 0 | 0 | 0 | 0.0 |
| 2 | 0 | 0 | 1 | 0.3 |
| 3 | 0 | 0 | 0 | 0.0 |
| 4 | 0 | 0 | 1 | 0.3 |
| 5 | 0 | 0 | 0 | 0.0 |
| 6 | 0 | 0 | 0 | 0.0 |
| Erythema Score | *0.0* | *0.0* | *0.3* | **0.1** |

In accordance with the interpretation of the DII showed in example 3, we conclude that the patch of the present invention produces negligible irritation.

Example 11: Bioavailability and dermal irritation evaluation of two different sizes of selegiline TDS according to the present invention, in humans.

The biovailability study was performed after a single dose in seven healthy male volunteers. Safety data (clinical and laboratory data, local reactions and systemic adverse events) and patch performance were also recorded, as secondary targets.

The trial was an open label, single dose, randomized, crossover pharmacokinetics study,which consisted in 2 periods of treatment of 24 h each, separated by a washout period of 14 days. The following formulations were tested, Selsyn 15 cm² (lot 80103) and Selsyn 30 cm² (lot 80102). Composition of the lots are shown in table 18

**Table 18: Composition of the lots used for bioavailability and dermal irritation evaluation**

| Component | Selsyn 15 cm2 (lot 80103) | Selsyn 30 cm2 (lot 80102) |
|---|---|---|
| Selegiline | 10.8 % | 10.8 % |
| DT 87-2353 | 83.6% | 83.6% |
| Squalene | 5.6 % | 5.6 % |

A baseline blood sample was obtained before patch application. During patch use, blood samples were drawn at the following times: 2h, 3h, 4h, 8h, 12h, 18 h and 24h. A final blood sample was collected at 48 hours.
(24 hours after patch removal).

Patch adhesion was evaluated throughout the study as well as dermal irritation, assessed 15-30 minutes following patch removal.

### Results

### a) Bioavailability

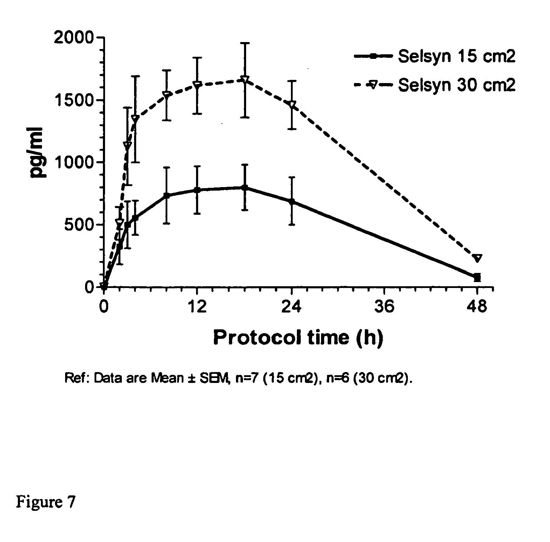

A proportional relationship was obtained between patch size and area under the curve for plasmatic selegiline (see Figure 7).

We conclude that we have obtained a selegiline transdermal device capable of delivering selegiline in a controlled fashion for 24 hours with a size dependant output.

### b) Irritation

No skin reaction in the area of application was found in 3 of 7 cases (43%) for SELSYN 15 cm2 and 6 of 7 cases with SELSYN 30 cm2 (86%). "Barely perceptive redness" (score 1) was observed in other 3 of 7 cases (43%) only during SELSYN 15 cm2 treatment. "Definite redness" (score 2) developed in 1 of 7 (14%) cases during both SELSYN 15 cm2 and SELSYN 30 cm2 treatments (different subject). The extent of such reactions, respect to the application area, ranged between 50% to 100%. None of the subjects complained about these findings.

None of the skin reactions observed throughout the study were diagnosed with grades 3 ("beet redness erythema") or 4 ("beet redness with slight eschar and/or blister formation").

The overall skin tolerability of the patch formulation according to the present invention can be considered as quite good.

### c) Adhesion

For adhesion evaluation the FDA classification was employed. Results are shown in Table 19:

**Table 19: Selsyn TDS adhesion profile**

| Formulation Tested | Transdermal System Adhesion | | | | | |
|---|---|---|---|---|---|---|
| | ≥90% adhered 1 | ≥75% to <90% adhered 2 | ≥50% to <75% adhered 3 | <50% adhered but not detached 4 | System detached 5 | Total Tested |
| 15 CM2 Lot 80103 | 7 | 0 | 0 | 0 | 0 | 7 |
| 30 CM2 Lot 80102 | 4 | 2 | 1 | 0 | 0 | 7 |

| | | | | | | |
|---|---|---|---|---|---|---|
| 1. essentially no detachments from the skin are observed 2. detachment of any of the patch borders 3. less than half of the patch detached from the skin 4. more than half of the patch detached from the skin, but without falling off 5. patch completely detached from the skin | | | | | | |

We conclude that adhesion performance was acceptable.

### References:

1) Am J Psychiatry 159:1869-1875, November 2002.
2) Primary Psychiatry 13(6):61-81, 2006.

## Claims

1. A monolithic device for transdermal administration of an active pharmaceutical ingredient which is liquid at 25°C, the device having an adhesive matrix layer which includes:
said active pharmaceutical ingredient which is selected from selegiline and rivastigmine,
an acrylic polymer pressure sensitive adhesive having a shear value of between 1.5 and 15 hours, and
a non-volatile coadjuvant selected from squalene and triethylcitrate present in the layer in an amount of 1 to 15wt%,
the adhesive matrix layer containing no component acting as a cross-linking agent for the acrylic polymer,
the device further having a backing film on a first face of the adhesive layer and a release liner on an opposite face of the adhesive layer.

2. A device according to claim 1, wherein the acrylic polymer pressure sensitive adhesive has functional groups selected from carboxyl (-COOH) and hydroxyl (-OH) functional groups.

3. A device according to claim 2, wherein the acrylic polymer pressure sensitive adhesive has carboxyl functional groups.

4. A device according to any one of claims 1 to 3, wherein the amount of the coadjuvant is in the range 2 to 15%.

5. A device according to any one of claims 1 to 3, wherein the adhesive matrix layer comprises:
selegiline as said active pharmaceutical ingredient, present at 8 - 12.5wt%, and
squalene as said non-volatile coadjuvant, present at 1 - 10wt%, and
said acrylic polymer pressure sensitive adhesive has a shear value in the range of 1.5 to 3 hours and has carboxyl functional groups.

6. A device according to claim 5, wherein the adhesive matrix layer comprises:
selegiline as said active pharmaceutical ingredient, present at 10 - 12.5wt%, and
squalene as said non-volatile coadjuvant, present at 2 - 6wt%.

7. A device according to any one of claims 1 to 3, wherein the adhesive matrix layer comprises:
rivastigmine as said active pharmaceutical ingredient, present at 12 - 30wt%, and
squalene as the non-volatile coadjuvant, present at 1 - 10wt%,
and wherein said acrylic polymer pressure sensitive adhesive has a shear value in the range of 1.5 to 3 hours and has carboxyl groups.

8. A device according to claim 7, wherein the adhesive matrix layer comprises:
rivastigmine as said active pharmaceutical ingredient, present at 16 - 30wt%, and
squalene as the non-volatile coadjuvant, present at 2 - 5wt%.

9. A device according to any one of claims 1 to 3, wherein the adhesive matrix layer comprises:
rivastigmine as said active pharmaceutical ingredient, present at 12 - 20wt%, and
squalene as the non-volatile coadjuvant, present at 1 - 10wt%,
and wherein said acrylic pressure sensitive adhesive has a shear value in the range of 1.5 to 3 hours and has carboxyl groups.

10. A method of manufacturing a monolithic device for transdermal administration of an active pharmaceutical ingredient which is liquid at 25°C, the device having an adhesive matrix layer which includes:
said active pharmaceutical ingredient which is selected from selegiline and rivastigmine,
an acrylic polymer pressure sensitive adhesive, having a shear value of between 1.5 and 15 hours, and a non-volatile coadjuvant selected from squalene and triethylcitrate present in the layer in an amount of 1 to 15wt%,
the adhesive matrix layer containing no component acting as a cross-linking agent for the acrylic polymer,
the method including the steps of:
compounding the active pharmaceutical ingredient, the pressure sensitive adhesive, and the non-volatile coadjuvant in order to obtain a homogeneous mixture;
casting said homogeneous mixture onto a release liner;
drying the release liner and homogeneous mixture at a temperature between 50 and 100°C, to form an adhesive layer on the release liner; and
applying a backing film to the adhesive layer.

11. A method according to claim 15, wherein said non-volatile coadjuvant is squalene.

12. A method according to claim 10 or 11, wherein the acrylic polymer pressure sensitive adhesive has carboxyl functional groups.

13. A method according to any one of claims 10 to 12, wherein the adhesive matrix layer comprises:
selegiline as said active pharmaceutical ingredient, present at 8 - 12.5wt%, and
squalene as said non-volatile coadjuvant, present at 1 - 10wt%, and
said acrylic polymer pressure sensitive adhesive has a shear value in the range of 1.5 to 3 hours and has carboxyl functional groups.

14. A method according to claim 13, wherein the adhesive matrix layer comprises:
selegiline as said active pharmaceutical ingredient, present at 10 - 12.5wt%, and
squalene as said non-volatile coadjuvant, present at 2 - 6wt%.

15. A method according to any one of claims 10 to 12, wherein the adhesive matrix layer comprises:
rivastigmine as said active pharmaceutical ingredient, present at 12 - 30wt%, and
squalene as the non-volatile coadjuvant, present at 1 - 10wt%,
and wherein said acrylic pressure sensitive adhesive has a shear value in the range of 1.5 to 3 hours and has carboxyl groups.

## Patentansprüche

1. Monolithische Vorrichtung zur transdermalen Verabreichung eines pharmazeutischen Wirkstoffs, der bei 25 °C flüssig ist, wobei die Vorrichtung eine Matrixklebeschicht aufweist, die Folgendes umfasst:
den pharmazeutischen Wirkstoff, der aus Selegilin und Rivastigmin ausgewählt ist,
einen Acrylpolymerhaftklebstoff mit einem Scherwert zwischen 1,5 und 15 h und
ein aus Squalen und Triethylcitrat ausgewähltes, nichtflüchtiges Coadjuvans, das in der Schicht in einer Menge von 1 bis 15 Gew.-% vorhanden ist,
wobei die Matrixklebeschicht keine Komponente enthält, die als Vernetzungsmittel für das Acrylpolymer wirkt,
und die Vorrichtung weiters einen Trägerfilm auf einer ersten Seite der Klebeschicht und eine Abziehschicht auf der entgegengesetzten Seite der Klebeschicht aufweist.

2. Vorrichtung nach Anspruch 1, worin der Acrylpolymerhaftklebstoff aus funktionellen Carboxyl- (-COOH-) und Hydroxyl- (-OH-) Gruppen ausgewählte funktionelle Gruppen aufweist.

3. Vorrichtung nach Anspruch 2, worin der Acrylpolymerhaftklebstoff funktionelle Carboxylgruppen aufweist.

4. Vorrichtung nach einem der Ansprüche 1 bis 3, worin die Menge des Coadjuvans im Bereich von 2 bis 15 % liegt.

5. Vorrichtung nach einem der Ansprüche 1 bis 3, worin die Matrixklebeschicht:
Selegilin als pharmazeutischen Wirkstoff in einer Menge von 8 bis 12,5 Gew.-% und
Squalen als nichtflüchtiges Coadjuvans in einer Menge von 1 bis 10 Gew.-% umfasst und
der Acrylpolymerhaftklebstoff einen Scherwert im Bereich von 1,5 bis 3 h und funktionelle Carboxylgruppen aufweist.

6. Vorrichtung nach Anspruch 5, worin die Matrixklebeschicht Folgendes umfasst:
Selegilin als pharmazeutischen Wirkstoff in einer Menge von 10 bis 12,5 Gew.-% und
Squalen als nichtflüchtiges Coadjuvans in einer Menge von 2 bis 6 Gew.-%.

7. Vorrichtung nach einem der Ansprüche 1 bis 3, worin die Matrixklebeschicht:
Rivastigmin als pharmazeutischen Wirkstoff in einer Menge von 12 bis 30 Gew.-% und
Squalen als nichtflüchtiges Coadjuvans in einer Menge von 1 bis 10 Gew.-% umfasst
und worin der Acrylpolymerhaftklebstoff einen Scherwert im Bereich von 1,5 bis 3 h und Carboxylgruppen aufweist.

8. Vorrichtung nach Anspruch 7, worin die Matrixklebeschicht Folgendes umfasst:
Rivastigmin als pharmazeutischen Wirkstoff in einer Menge von 16 bis 30 Gew.-% und
Squalen als nichtflüchtiges Coadjuvans in einer Menge von 2 bis 5 Gew.-%.

9. Vorrichtung nach einem der Ansprüche 1 bis 3, worin die Matrixklebeschicht:
Rivastigmin als pharmazeutischen Wirkstoff in einer Menge von 12 bis 20 Gew.-% und
Squalen als nichtflüchtiges Coadjuvans in einer Menge von 1 bis 10 Gew.-% umfasst
und worin der Acrylhaftklebstoff einen Scherwert im Bereich von 1,5 bis 3 h und Carboxylgruppen aufweist.

10. Verfahren zur Herstellung einer monolithischen Vorrichtung zur transdermalen Verabreichung eines pharmazeutischen Wirkstoffs, der bei 25 °C flüssig ist, wobei die Vorrichtung eine Matrixklebeschicht aufweist, die Folgendes umfasst:
den pharmazeutischen Wirkstoff, der aus Selegilin und Rivastigmin ausgewählt ist,
einen Acrylpolymerhaftklebstoff mit einem Scherwert zwischen 1,5 und 15 h und ein aus Squalen und Triethylcitrat ausgewähltes, nichtflüchtiges Coadjuvans, das in der Schicht in einer Menge von 1 bis 15 Gew.-% vorhanden ist,
wobei die Matrixklebeschicht keine Komponente enthält, die als Vernetzungsmittel für das Acrylpolymer wirkt,
und das Verfahren die folgenden Schritte umfasst:
das Vermischen des pharmazeutischen Wirkstoffs, des Haftklebstoffs und des nichtflüchtigen Coadjuvans, um ein homogenes Gemisch zu erhalten;
das Gießen des homogenen Gemischs auf eine Abziehschicht;
das Trocknen der Abziehschicht und des homogenen Gemischs bei einer Temperatur zwischen 50 und 100 °C, um eine Klebeschicht auf der Abziehschicht zu bilden; und
das Auftragen eines Trägerfilms auf die Klebeschicht.

11. Verfahren nach Anspruch 10, worin das nichtflüchtige Coadjuvans Squalen ist.

12. Verfahren nach Anspruch 10 oder 11, worin der Acrylpolymerhaftklebstoff funktionelle Carboxylgruppen aufweist.

13. Verfahren nach einem der Ansprüche 10 bis 12, worin die Matrixklebeschicht:
Selegilin als pharmazeutischen Wirkstoff in einer Menge von 8 bis 12,5 Gew.-% und
Squalen als nichtflüchtiges Coadjuvans in einer Menge von 1 bis 10 Gew.-% umfasst und
der Acrylpolymerhaftklebstoff einen Scherwert im Bereich von 1,5 bis 3 h und funktionelle Carboxylgruppen aufweist.

14. Verfahren nach Anspruch 13, worin die Matrixklebeschicht Folgendes umfasst:
Selegilin als pharmazeutischen Wirkstoff in einer Menge von 10 bis 12,5 Gew.-% und
Squalen als nichtflüchtiges Coadjuvans in einer Menge von 2 bis 6 Gew.-%.

15. Verfahren nach einem der Ansprüche 10 bis 12, worin die Matrixklebeschicht:
Rivastigmin als pharmazeutischen Wirkstoff in einer Menge von 12 bis 30 Gew.-% und
Squalen als nichtflüchtiges Coadjuvans in einer Menge von 1 bis 10 Gew.-% umfasst
und worin der Acrylhaftklebstoff einen Scherwert im Bereich von 1,5 bis 3 h und Carboxylgruppen aufweist.

## Revendications

1. Dispositif monolithique pour l'administration transdermique d'un ingrédient pharmaceutique actif qui est liquide à 25°C, le dispositif ayant une couche de matrice adhésive qui comporte:
ledit ingrédient pharmaceutique actif qui est sélectionné parmi sélégiline et rivastigmine,
un adhésif de polymère acrylique réagissant à la pression ayant une valeur de cisaillement comprise entre 1,5 et 15 heures, et
un coadjuvant non volatile sélectionné parmi squalène et triéthylcitrate présent dans la couche en une quantité de 1 à 15% en poids,
la couche de matrice adhésive ne contenant pas de composant agissant comme un agent de réticulation pour le polymère acrylique,
le dispositif comportant en outre un film de support sur une première face de la couche adhésive et une doublure de relâchement sur une face opposée de la couche adhésive.

2. Dispositif selon la revendication 1, dans lequel l'adhésif en polymère acrylique réagissant à la pression a des groupes fonctionnels sélectionnés parmi des groupes fonctionnels carboxyle (-COOH) et hydroxyle (-OH).

3. Dispositif selon la revendication 2, dans lequel l'adhésif en polymère acrylique réagissant à la pression a des groupes carboxyles fonctionnels.

4. Dispositif selon l'une quelconque des revendications 1 à 3, dans lequel la quantité de coadjuvant est dans la plage de 2 à 15%.

5. Dispositif selon l'une quelconque des revendications 1 à 3, dans lequel la couche de matrice adhésive comprend:
de la sélégiline comme ingrédient pharmaceutique actif, présent à 8-12,5% en poids, et
du squalène en tant que coadjuvant non volatile précité, présent à 1-10% en poids, et
ledit adhésif en polymère acrylique réagissant à la pression a une valeur de cisaillement dans la plage de 1,5 à 3 heures et a des groupes carboxyles fonctionnels.

6. Dispositif selon la revendication 5, dans lequel la couche de matrice adhésive comprend:
de la sélégiline comme ingrédient pharmaceutique actif, présent à 10-12,5% en poids, et
du squalène comme coadjuvant non volatile précité, présent à 2-6% en poids.

7. Dispositif selon l'une quelconque des revendications 1 à 3, dans lequel la couche de matrice adhésive comprend:
de la rivastigmine comme ingrédient pharmaceutique actif précité, présent à 12-30% en poids, et
du squalène comme coadjuvant non volatile, présent à 1-10% en poids,
et où ledit adhésif en polymère acrylique réagissant à la pression a une valeur de cisaillement dans la plage de 1,5 à 3 heures et possède des groupes carboxyles.

8. Dispositif selon la revendication 7, dans lequel la couche de matrice adhésive comprend:
de la rivastigmine comme ingrédient pharmaceutique actif précité, présent à 16-30% en poids, et
du squalène comme coadjuvant non volatile, présent à 2-5% en poids.

9. Dispositif selon l'une quelconque des revendications 1 à 3, dans lequel la couche de matrice adhésive comprend:
de la rivastigmine comme ingrédient pharmaceutique actif précité, présent à 12-20% en poids, et
du squalène comme coadjuvant non volatile, présent à 1-10% en poids,
et où ledit adhésif en polymère acrylique réagissant à la pression a une valeur de cisaillement dans la plage de 1,5 à 3 heures et possède des groupes carboxyles.

10. Procédé de fabrication d'un dispositif monolithique pour l'administration transdermique d'un ingrédient pharmaceutique actif qui est liquide à 25°C, le dispositif comportant une couche de matrice adhésive qui comporte:
ledit ingrédient pharmaceutique actif qui est sélectionné parmi sélégiline et rivastigmine,
un adhésif en polymère acrylique réagissant à la pression, ayant une valeur de cisaillement comprise entre 1,5 et 15 heures et un coadjuvant non volatile sélectionné parmi squalène et triéthylcitrate présent dans la couche en une quantité de 1 à 15% en poids,
la couche de matrice adhésive ne contenant pas de composant agissant comme agent de réticulation pour le polymère acrylique,
la méthode incluant les étapes de:
le compoundage de l'ingrédient pharmaceutique actif, de l'adhésif réagissant à la pression et du coadjuvant non volatile pour obtenir un mélange homogène;
la coulée dudit mélange homogène sur une doublure de relâchement;
le séchage de la doublure de relâchement et du mélange homogène à une température entre 50 et 100°C, pour former une couche adhésive sur la doublure de relâchement; et
l'application d'un film de support à la couche adhésive.

11. Procédé selon la revendication 15, dans lequel ledit coadjuvant non volatile est le squalène.

12. Procédé selon la revendication 10 ou 11, dans lequel l'adhésif en polymère acrylique réagissant à la pression a des groupes carboxyles fonctionnels.

13. Procédé selon l'une quelconque des revendications 10 à 12, dans lequel la couche de matrice adhésive comprend:
de la sélégiline comme ingrédient pharmaceutique actif, présent à 8-12,5% en poids, et
du squalène comme coadjuvant non volatile précité, présent à 1-10% en poids, et
ledit adhésif en polymère acrylique réagissant à la pression à une valeur de cisaillement dans la plage de 1,5 à 3 heures et possède des groupes fonctionnels carboxyles.

14. Procédé selon la revendication 13, dans lequel la couche de matrice adhésive comprend:
de la sélégiline comme ingrédient pharmaceutique actif, présent à 10-12,5% en poids, et
du squalène comme coadjuvant non volatile précité, présent à 2-6% en poids.

15. Procédé selon l'une quelconque des revendications 10 à 12, dans lequel la couche de matrice adhésive comprend:
de la rivastigmine comme ingrédient pharmaceutique actif précité, présent à 12-30% en poids, et
du squalène comme coadjuvant non volatile, présent à 1-10% en poids,
et où ledit adhésif acrylique réagissant à la pression a une valeur de cisaillement dans la plage de 1,5 à 3 heures et possède des groupes carboxyles.
